# EUROPEAN PATENT SPECIFICATION

(11) **EP 2 331 128 B1**
(45) Date of publication and mention of the grant of the patent: **06.11.2013**
(21) Application number: 09782654.9
(22) Date of filing: 04.09.2009
(51) Int. Cl.: A61K 39/39, A61K 39/17, A61P 31/12

(54) **COMPOSITION COMPRISING CHITOSAN FOR OCULAR ADMINISTRATION OF VACCINE(S) TO AVIANS**
ZUSAMMENSETZUNG MIT CHITOSAN ZUR OKULAREN VERABREICHUNG VON IMPFSTOFFEN BEI VÖGELN
COMPOSITION COMPRENANT DU CHITOSANE POUR ADMINISTRATION OCULAIRE DE VACCIN(S) À DES AVIAIRES

(30) Priority: 05.09.2008 US 94642 P; 03.04.2009 US 166398 P
(43) Date of publication of application: 15.06.2011
(73) Proprietor: CEVA Santé Animale SA, 33501 Libourne Cedex (FR)
(72) Inventor: GARDIN, Yannick, F-49460 Cantenay Epinard (FR); PALYA, Vilmos, H-1223 Budapest (HU); COMTE, Sylvain, F-33370 Salleboeuf (FR)
(74) Representative: Becker, Philippe
(86) International application number: PCT/EP2009/061508
(87) International publication number: WO 2010/026239

(56) References cited:
- WO-A2-2004/075829
- DEGEN W G J ET AL: "Potentiation of humoral immune responses to vaccine antigens by recombinant chicken IL-18 (rChIL-18)" VACCINE, BUTTERWORTH SCIENTIFIC. GUILDFORD, GB, vol. 23, no. 33, 21 July 2005 (2005-07-21) , pages 4212-4218, XP004973229 ISSN: 0264-410X
- REHMANI SHAFQAT F: "Newcastle disease vaccination: A comparison of vaccines and routes of administration in Pakistan" PREVENTIVE VETERINARY MEDICINE, vol. 25, no. 3-4, 1996, pages 241-248, XP002555808 ISSN: 0167-5877
- BOX P G ET AL: "IMMUNISATION OF MATERNALLY IMMUNE TURKEY POULTS AGAINST NEWCASTLE DISEASE", AVIAN PATHOLOGY, HUNTINGDON, CAMBS, GB, vol. 5, no. 4, 1 January 1976 (1976-01-01) , pages 307-314, XP008039070, ISSN: 0307-9457

## Description

### Field of the invention

The invention relates to vaccine compositions comprising chitosan and a vaccine which are administered to avian species via the ocular route, and methods or programs of vaccination including use of chitosan in order to increase or enhance immune response to vaccine in avian species.

### Background of the invention

Vaccines are preparations of antigenic materials, administered to a subject in order to enhance resistance to infection by inducing active immunity to specific microorganisms, such as bacteria or viruses or parasites. Vaccines may be co-administered with adjuvants in order to boost the immune response. Examples of adjuvants include chemical and polypeptide immunostimulants which enhance the immune system response to antigens. Such adjuvants may include for example, aluminum hydroxide, aluminum phosphate, plant and animal oils as well as mineral oils, bacterial toxins, chitin derivatives and chitosan and the like which are administered with the vaccine in an amount sufficient to enhance the immune response.

Vaccines and adjuvants are generally administered via parenteral injections, such as subcutaneous or intramuscular injections. Direct administration onto the nasal mucosa of various animal species may be also performed especially for treating infections of the upper respiratory tract. For example, the European patent EP 865 297 B1 describes intranasal coadministration to mice of protein or polysaccharide antigens from pathogens and chitosan as adjuvant. These intranasal compositions are formulated as dry powders in the form of microspheres, via aerosols, drops or insufflations, and allow enhancing both IgA mucosal humoral and IgG systemic immune responses further several successive intranasal administrations. Rehmani S.F., (Preventive Veterinary Medicine 25 (1996), 241-248) discloses a vaccine composition comprising live attenuated Newcastle Disease Virus for immunisation by ocular administration.

The Applicant has now discovered that chitosan used in combination with an avian vaccine within a same vaccination program has the potential of greatly enhancing cell-mediated immune response, while maintaining high humoral and systemic immune responses of vaccinated birds when administered via the ocular route. In addition, it has been discovered that chitosan was acting on the avian conjunctiva and resulted in a significant increase of the persistence of the vaccine within the avian tissues.

### Summary of the invention

The present invention relates to vaccine compositions for ocular administration to avian species, as defined in the claims. The invention also relates to uses of NDV in combination with chitosan for the manufacture of a vaccine composition for ocular administration to efficiently immunize avian species against infectious diseases. The present invention further relates to an avian vaccination kit as defined in the claims.

### Brief description of the Figures

**FIGURE 1****:** illustrates the timeline of vaccination.
**FIGURE 2****:** illustrates the antigen recall protocol.
**FIGURE 3****:** shows the levels of production of chicken γ-interferon (Optic Density) as measured 2 to 5 weeks after ocular administration of CEVAC^{®} VITAPEST L (Ceva Phylaxia, Hungary) with or without chitosan.
**FIGURE 4****:** shows the titers of Newcastle disease (ND) vaccinal strain expressed in egg infection dose (EID₅₀) within avian tissues of SPF chickens which have received CEVAC^{®} UNI L (Ceva Phylaxia, Hungary), or CEVAC^{®} VITAPEST L, with or without chitosan via the ocular route.
**FIGURE 5****:** shows the titers of Newcastle disease vaccine strain expressed in egg infection dose (EID₅₀) within avian tissues of conventional layer chickens which have received CEVAC^{®} UNI L, or CEVAC^{®} VITAPEST L, with or without chitosan via the ocular route.

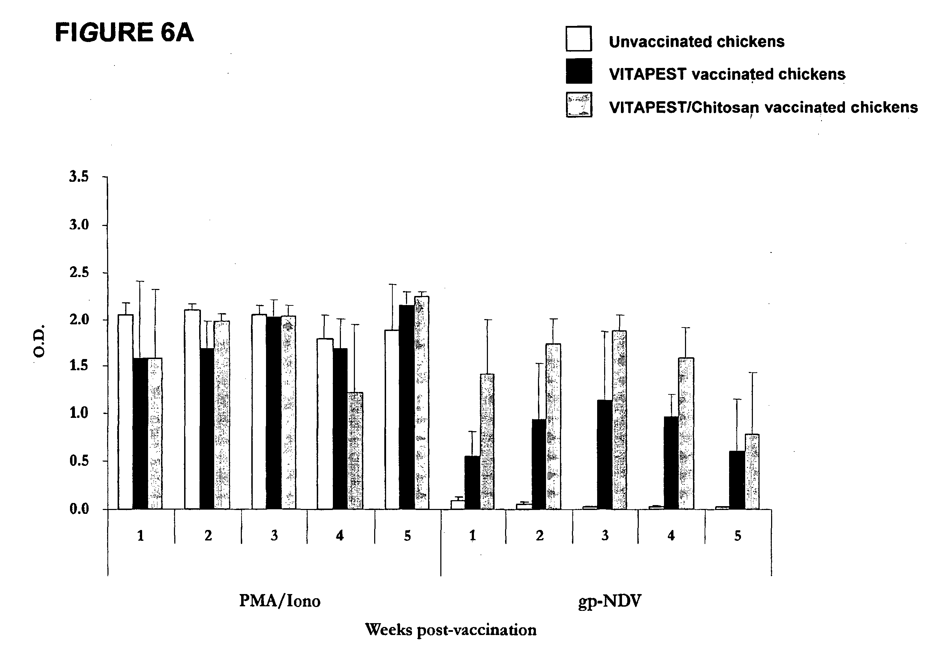

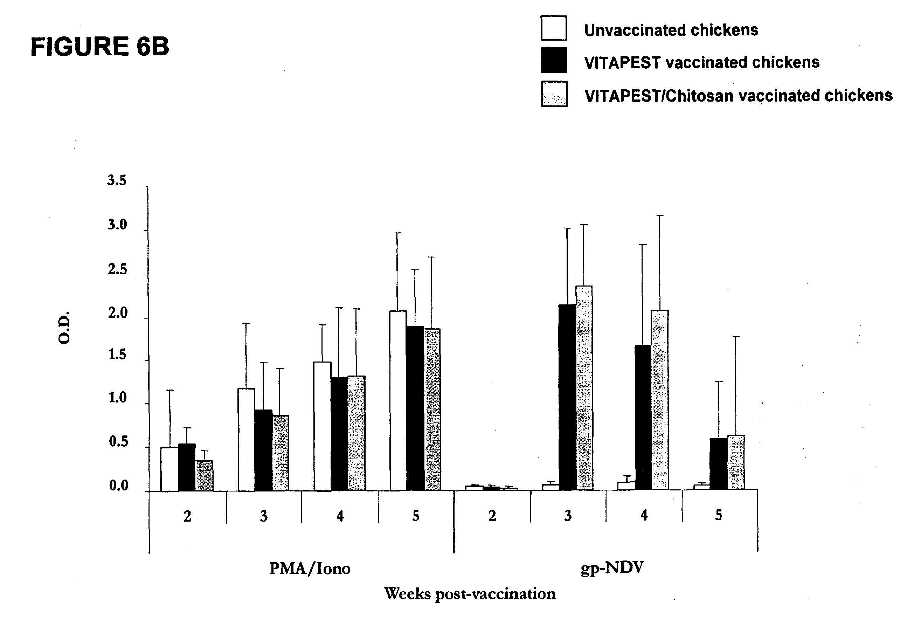

**FIGURES 6A** **and** **6B****:** show immunocompetence and specific cell-mediated immunity after vaccination of SPF (Figure 6A) and conventional layer (Figure 6B) chickens with live Newcastle disease vaccine (NDV). Birds were vaccinated at day-old, via ocular route, with CEVAC^{®} VITAPEST L alone (black columns) or in combination with chitosan (grey columns). Unvaccinated animals are indicated by white columns. Splenocytes were stimulated with PMA/lono mitogen (0.1 µg/ml) gp-NDV recall antigen (1 µg/ml) and supernatants of stimulated cells were harvested after 72 h of activation. ChIFNγ production was determined by the ChIFNγ capture ELISA. The results correspond to the mean ± standard deviation of optical density (O.D.) at each time point (n = 5). Means ± standard deviations with no common superscript differ significantly (*P* < *0,05*).

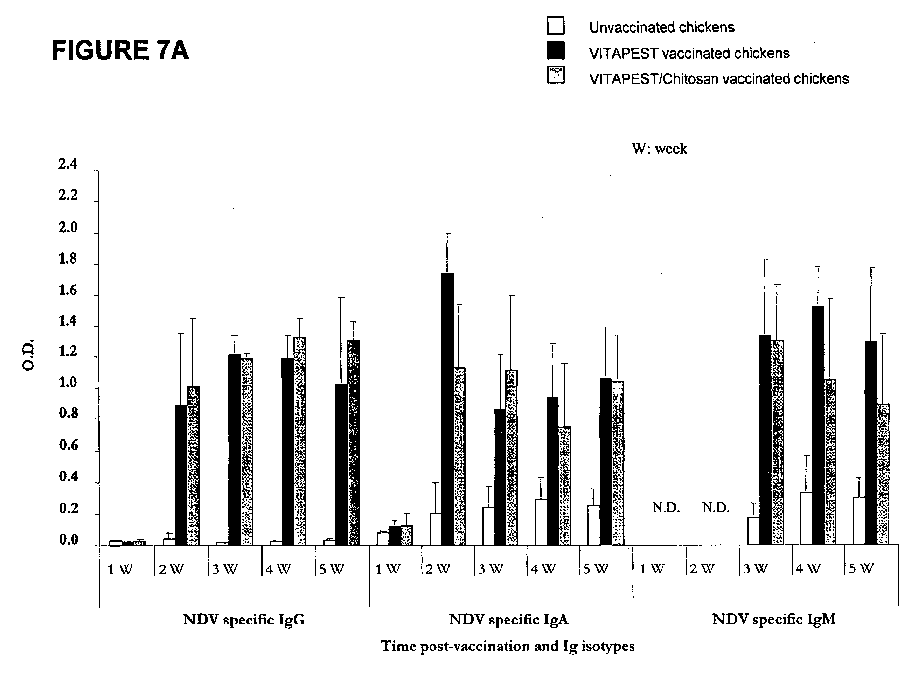

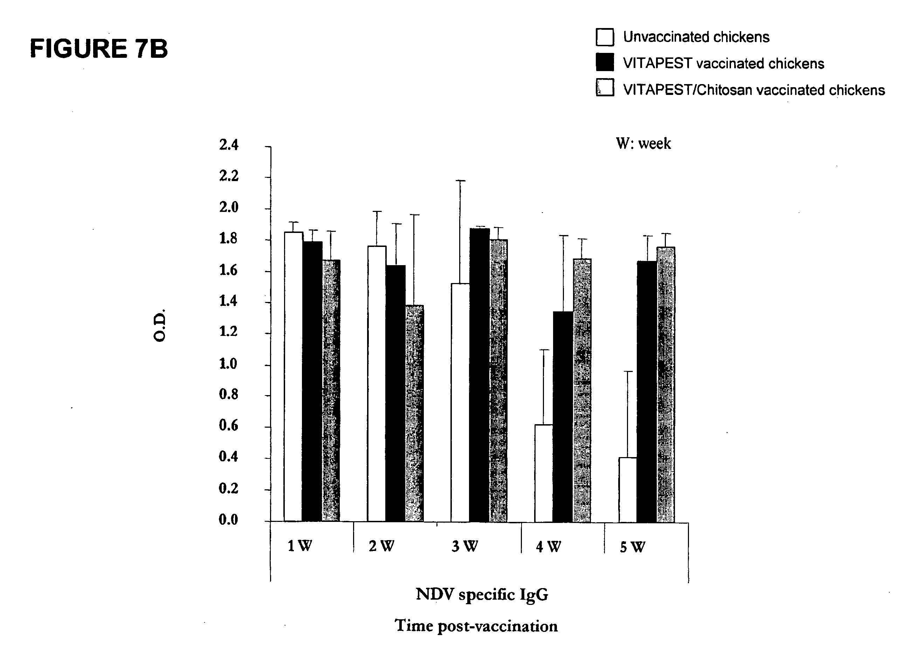

**FIGURES 7A** **and** **7B****:** show lachrymal antibody-mediated immunity after vaccination of SPF (Figure 7A) and conventional layer (Figure 7B) chickens with live ND vaccine. Birds were vaccinated at one day-old , via ocular route, with CEVAC^{®} VITAPEST L alone (black columns) or in combination with chitosan (grey columns). Unvaccinated animals are indicated by white columns. Data represent mean ± standard deviation of absorbance values (O.D.) determined by ELISA at specified time post-vaccination (n = 5). Tears samples were diluted 1:4 for IgA/G and 1:32 for IgM. Mean ± standard deviation at time points with no common superscript differ significantly (*P < 0.05*). N.D. = not determined, due to limited amount of samples left following NDV specific IgA and IgG measurements.

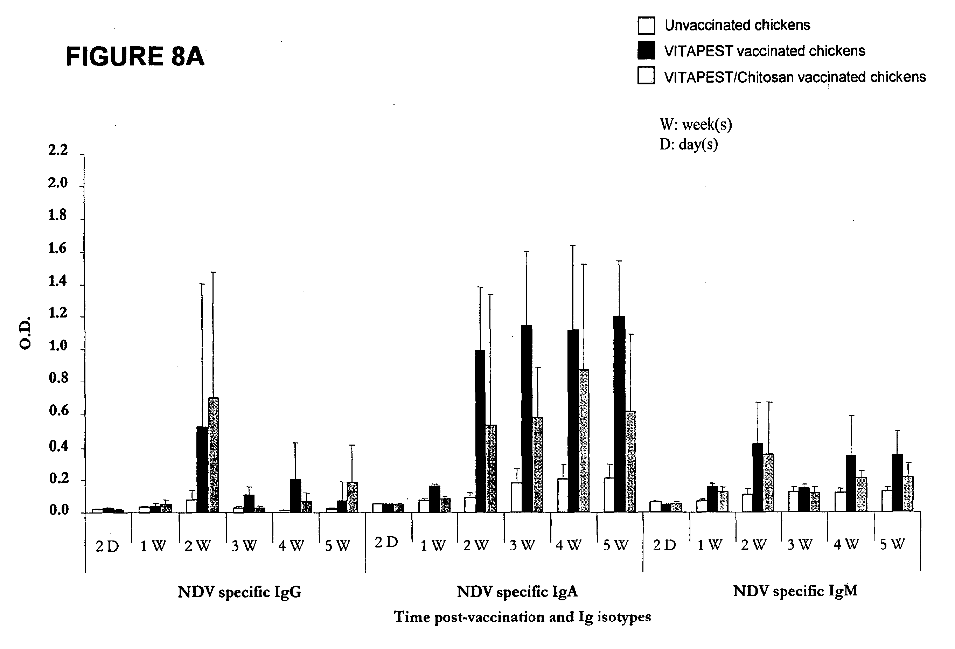

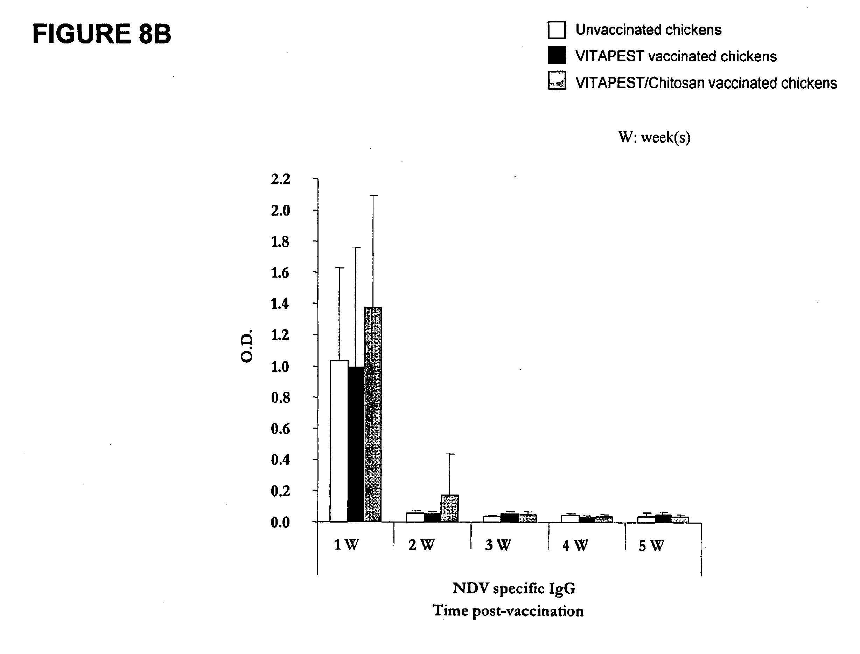

**FIGURES 8A** **and** **8B****:** show biliary antibody-mediated immunity after vaccination of SPF (Figure 8A) and conventional layer (Figure 8B) chickens with live ND vaccine. Birds were vaccinated at one day-old , via ocular route, with CEVAC^{®} VITAPEST L alone (black columns) or in combination with chitosan (grey columns). Unvaccinated animals are indicated by white columns. Data represent mean ± standard deviation of absorbance values (O.D.) determined by ELISA at specified time post-vaccination (n = 5). Bile samples were diluted 1:50. Mean ± standard deviation at time points with no common superscript differ significantly (*P < 0.05*).

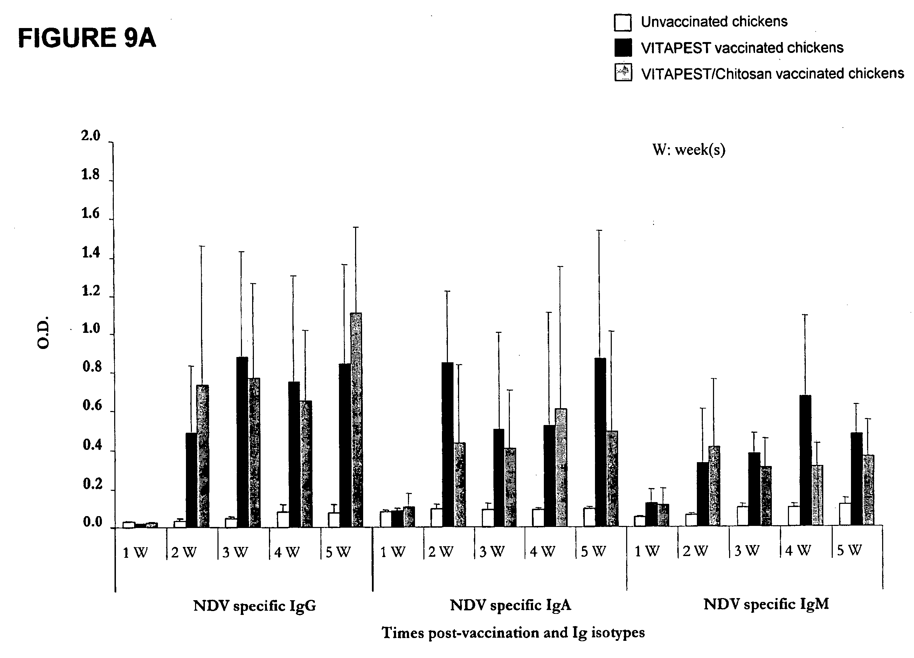

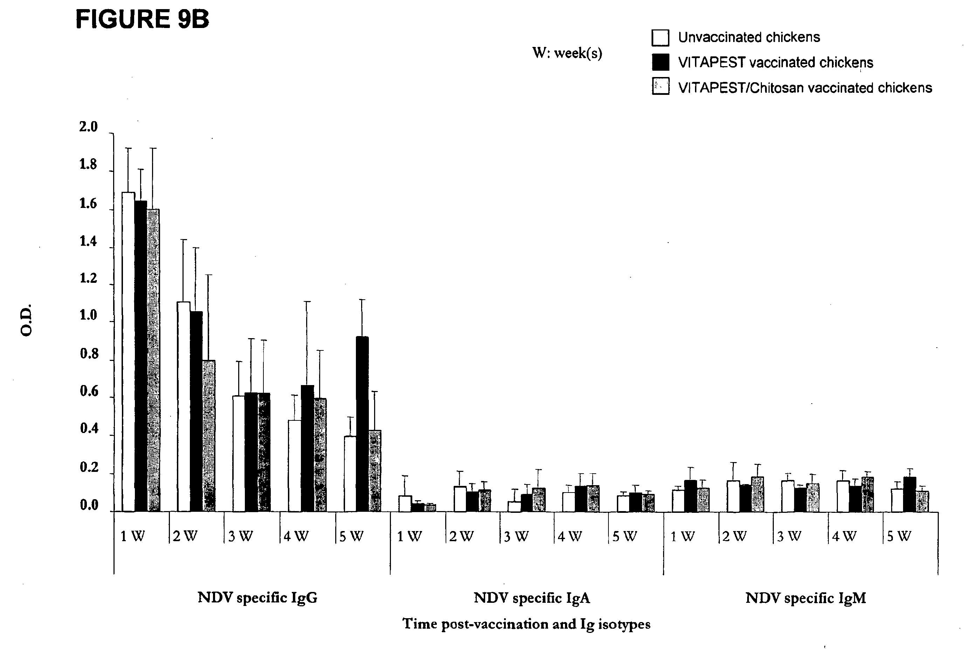

**FIGURES 9A** **and** **9B****:** show duodenal antibody-mediated immunity after vaccination of SPF (Figure 9A) and conventional layer (Figure 9B) chickens with live ND vaccine. Birds were vaccinated at one day-old with CEVAC^{®} VITAPEST L alone (black columns) or in combination with chitosan (grey columns). Unvaccinated animals are indicated by white columns. Data represent mean ± standard deviation of absorbance values (O.D.) determined by ELISA at specified time post-vaccination (n = 5). The Ig response was measured in undiluted supernatants of *ex vivo* duodenal tissues cultures. Mean ± standard deviation at time points with no common superscript differ significantly (*P < 0.05*).

### Detailed description of the invention

The present invention discloses a vaccine composition for ocular administration to avian species, which composition comprises one or more vaccine and an effective adjuvant amount of chitosan. As mentioned above, it has been surprisingly found that, upon ocular co-administration, chitosan and a vaccine greatly enhances cell-mediated immune response of avian species and results in a longer persistence of the vaccine in specific tissues of the vaccinated birds. The vaccine compositions disclosed in the present invention are thus particularly effective for improving long term protection of avian species against avian diseases.

The term "effective adjuvant amount" is be well understood by those skilled in the art, and includes an amount of a chitosan which is capable of stimulating the immune response against the ocularly administered antigens, *i.e*., an amount that increases the immune response of an ocularly administered vaccine composition, for example as measured in terms of the levels of production of γ-interferon by splenocytes and persistence of vaccine in avian tissues. Significant increases in γ-interferon levels of production and vaccine tissues persistence were observed in presence of chitosan. By way of example, increase of cell response may also be evidenced by cell proliferation test (measured by incorporation of ³H thymidine into dividing cells after antigen recall stimulation), by measuring cytotoxic activity of CTL cells on radiolabelled target cells (determination of the release of radioisotope over a period of time), or via a macrophages migration test.

A vaccine may be any avian pathogenic or not microorganisms, such as viruses, bacteria, any other parasites, or antigens. These may be live attenuated microorganisms or killed inactivated microorganisms, either whole microorganisms or microorganisms' subunits, inactivated chimeric or recombinant microorganisms, disrupted microorganisms, mutant microorganisms, defective microorganisms, or combinations thereof. The vaccine may also be an antigen including epitopes or antigenic parts of the microorganism structure, *e.g.*, virus, bacteria or parasite, such as preparations of antigenic proteins from pathogens, recombinant proteins, preferably viral antigen, such as viral capsid proteins, cell wall proteins, peptides, or parts of bacterial or parasite structure, such as polysaccharides, lipopolysaccharides and glycoproteins. The vaccine may also be a DNA or recombinant DNA which produces the antigen from pathogens into cells after introduction of the DNA. Antigens may be provided in a purified or an unpurified form.

When the vaccine is a live attenuated (replicative) microorganism, such as virus, bacteria or other avian pathogen, the attenuated pathogen retains non pathogenic properties, and is capable of replication. Attenuation can come from natural or artificial attenuation process. Artificial attenuation is generally obtained using various techniques including passages in living animals or various natural media including organs, cells, embryonated eggs, etc. Artificial attenuation can also be obtained by drying of infected organs, aging of cultures, adaptation to low temperatures or particular conditions of culture, genetic deletions, etc...

A vaccine may also be killed inactivated microorganisms. Preparation of inactivated viruses for vaccination is generally achieved via chemical or physical means. Chemical inactivation can be effected by treating viruses for example with enzymes, formaldehyde, β-propiolactone, ethylene-imine or a derivative thereof. Inactivated virus so obtained may be neutralized or stabilized afterwards. Physical inactivation may be carried out by subjecting viruses to energy-rich radiation, such as UV-light, X-radiation or γ-radiation.

Such attenuated or inactivated microorganisms, *e.g*., viruses, bacteria or other avian parasites may also be purchased from commercial sources.

Vaccine may be homologous (such as chicken virus to protect chickens) or heterologous (such as turkey virus to protect chickens) types. Alternatively, vaccine preparations may comprise a combination of live antigens with specific antibodies, called immuno complex vaccines.

According to the present invention, the vaccine or antigen is derived from the Newcastle Disease virus (NDV).

Chitosan is a derivative of chitin and corresponds to a linear polysaccharide composed of randomly distributed β-(1-4)-linked D-glucosamine (deacetylated unit) and N-acetyl-D-glucosamine (acetylated unit). Chitosan is produced commercially by deacetylation of chitin, which is the structural element in the exoskeleton of crustaceans. The degree of deacetylation can be determined by NMR spectroscopy. For example, degree of deacetylaton of commercial chitosan is in the range 60-100 %.

Chitosan used in the vaccine compositions of the present invention may be produced from chitin by deacetylation to a degree of greater than 40%, preferably between 50% and 90%, and more preferably between 70% and 95%, deacetylation. Such deacetylated chitosans are commercially available under the commercial designation "Sea Cure⁺" chitosan glutamate from Protan Biopolymer A/S, Drammen, Norway. The molecular weight of the chitosan may be between 10 kD and 500 kD, preferably between 50 kD and 300 kD and more preferably between 100 kD and 300 kD.

According to the present invention, chitosan may be used as effective adjuvant in the form of Mono Carboxy Methylated Chitosan (MMC) also designated Carboxy Methyl Chitosan (CMC) or in the form of Hydroxychloride Chitosan (HCl Chitosan). HCl Chitosan is well known in the art and is marketed for example by Kraeber Gmbh & Co.

For example, concentrations of HCl Chitosan used in the vaccine compositions may be in the range 0.1% to 5%, and preferably around 0.5% or around 1%.

Vaccine compositions according to the invention may be used in the effective immunization of avian species against infectious avian diseases. In effect, as mentioned above, it has been found that upon ocular administration, these vaccine compositions greatly enhanced cell-mediated immune response of avians as well as persistence of vaccine in numerous avian tissues. Indeed, persistence of vaccine in avian tissues was observed one week after vaccination, in avian tissues including conjunctiva, lungs, spleen, trachea, digestive tract such as duodenum, pancreas and caecal tonsils. Furthermore, such persistence was maintained five weeks after vaccination, particularly in conjunctiva, lungs, duodenum, pancreas and caecal tonsils.

Avian species which may be ocularly administered with the vaccine compositions of the present invention include for example chickens, turkeys, geese, ducks, pheasants, quail, or pigeons and more generally raised or domestic poultry or fowl.

The invention discloses a use of vaccine compositions as defined herein in a method of vaccinating avian against avian infections, which method comprises ocularly administering to an avian subject a vaccine composition comprising a vaccine together with an effective adjuvant amount of chitosan.

The present invention also discloses the use of an effective amount of chitosan and a live attenuated Newcastle Disease Virus for the preparation of a vaccine composition for increasing avian immune response against an avian pathogen wherein the chitosan and a vaccine are administered to avians via eye-drops, sprays or aerosols, and wherein cell mediated response of avians and persistence of the pathogen within avian tissues are increased. The vaccine is a live attenuated Newcastle Disease Virus. Also, chitosan and the vaccine may be administered separately or simultaneously to avian species. When the chitosan and the pathogen are administered sequentially, chitosan may then be administered prior to the vaccine or subsequently to the vaccine.

Moreover, according to a further aspect the invention, provides a vaccine composition as defined herein for use in a method of enhancing protective cell-mediated immune response and persistence of the vaccine in avian tissues by ocular administration to avian species of a vaccine composition comprising a NDV and an effective adjuvant amount of chitosan as hereinbefore defined.

The present invention further discloses a method of immunizing avian species comprising a step of administering into the eyes of avian species the vaccine composition as described above. The present invention also discloses a method of increasing cell-mediated immunity in avian species and persistence of the vaccine within avian tissues by administering one or more vaccine in combination with an effective amount of chitosan into the eyes of avian species. The vaccine and chitosan may be administered separately, sequentially or simultaneously.

Also, according to the method of the present invention, ocular administration may be performed via spraying, aerosol or eye drop. Preferably, such ocular administration is performed after maternal-derived antibody waning.

Most preferably, a boost of vaccination is further conducted in order to stimulate the immune response of avians which have already been subject to vaccination in ovo or at few days of age thereby inducing a higher stimulation of the immune response. Such boost of vaccination may be conducted of 1 day to 4 weeks of age, either subsequently or concomitantly to the first immunization.

The ocular vaccine compositions according to the invention can be formulated as liquids or dry powders, for administration as aerosols, sprays or eye-drops. Vaccine compositions may thus be administered to avians by spraying directly onto the heads of the birds. Afternatively, one or more drops of the vaccine composition may be placed directly into the eyes of each individual bird as for humans. Preferred compositions according to the invention are formulated as eye-drops in the form of liquids.

Compositions for administration as aerosols, eye-drops or sprays may contain one or more excipients of the type usually included in such compositions, for example preservatives, viscosity adjusting agents, tonicity adjusting agents, lachrymal blocker agents, buffering agents, stabilizers, carriers, adjuvants, and the like for the preparation of ocular administration. In order to ensure that the chitosan remains soluble in an aqueous medium, and to ensure also that the antigen is not adversely affected by too acidic a pH, a solution for ocular administration preferably has a pH in the range 5.5 to 6.5. Carriers may be any of a number of aqueous buffers well known to those skilled in the art, such as for example phosphate buffers. Additional adjuvants may be used. These are well known in the art and may include oil-emulsions, aluminium salts or gels, such as aluminium hydroxide or aluminium phosphate, saponins, vitamins, extracts from bacterial wall, polymers based on polyacrylic acid, such as carbopols, non ionic block polymers, fatty acid amines, such as avridin and DDA, polymers based on dextran, such as dextran sulphate and DEAE dextran, biodegradable microcapsules, liposomes, viral immune stimulators, such as MDP, LPS, and glucans.

The present invention also discloses a product or an avian vaccination kit comprising a means for dispensing the ocular vaccine compositions comprising a vaccine and an effective amount of chitosan. More precisely, the avian vaccination kit comprises a dispensing device adapted for dispensing the vaccine composition directly into the eyes of the birds. Such dispensing device may, for example, take the form of an aerosol, a spray or eyedrop delivery system, and may be arranged to dispense only a single dose, or a multiplicity of doses into the eyes of avians. The avian vaccination kit may comprise separate containers respectively comprising an effective amount of chitosan and of the vaccine. The avian vaccination kit according to the present invention may further comprise technical instructions with information on the administration and dosage of the pharmaceutical composition.

The vaccine may be administered in an amount effective to stimulate cell-mediated immune response and to increase persistence of vaccine, such as for example of the vaccine virus strain in avian species. For example, the vaccine may be administered to avians in one or more doses, each dose containing for example 10⁵ to 10⁸ EID₅₀.

Current live vaccines are usually limited due to the presence in newly hatched birds of some protection against pathogens provided by the transmission of maternal-derived antibody (MDA), transferred via the egg yolk and subsequently resorbed and present in the chick serum. These MDA can interfere with vaccination by neutralizing the live vaccines. As a consequence, the immune response to vaccination may be delayed and/or limited in conventional chickens, as previously observed at systemic level.

Vaccines according to the present invention are thus preferably administrated after MDA has waned, thereby allowing for the induction of a good immunological response before the birds are likely to be exposed to a virulent strain of NDV. Most preferably, a boost vaccination is conducted at 2 or 3 weeks of age according to the decline of MDA.

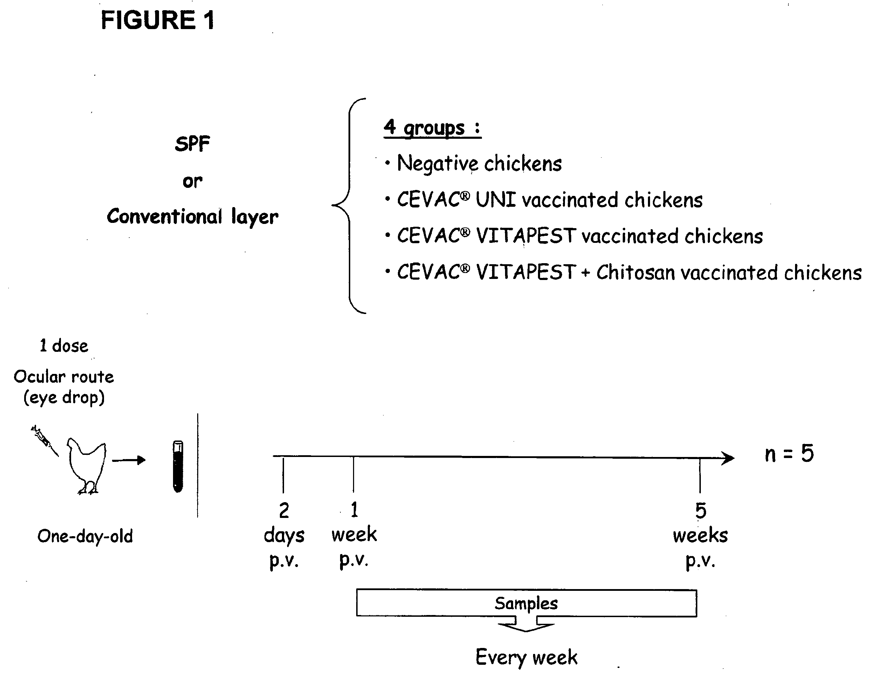

As showed with more details in the Examples below, vaccination experiments were conducted on four groups of SPF (Specific Pathogen Free) or conventional layer one day old chickens (Figure 1). Vaccine compositions were administered to all groups of chickens via the ocular route using eye drops. The group designated "Negative chickens" did not receive any vaccine. Second and third groups were vaccinated with one dose of CEVAC^{®} UNI L or with one dose of CEVAC^{®} VITAPEST L which both correspond to live attenuated Newcastle Disease virus. The chitosan was also used as adjuvant. Only negative and CEVAC^{®} VITAPEST L were vaccinated with or without adjuvant to confirm the surprising chitosan effect both on the cell-mediated immunity and persistence of the vaccine virus in avian tissues. Samples were collected each week from the first to the fifth week. Assessment and confirmation of local (BALT) immunity was conducted.

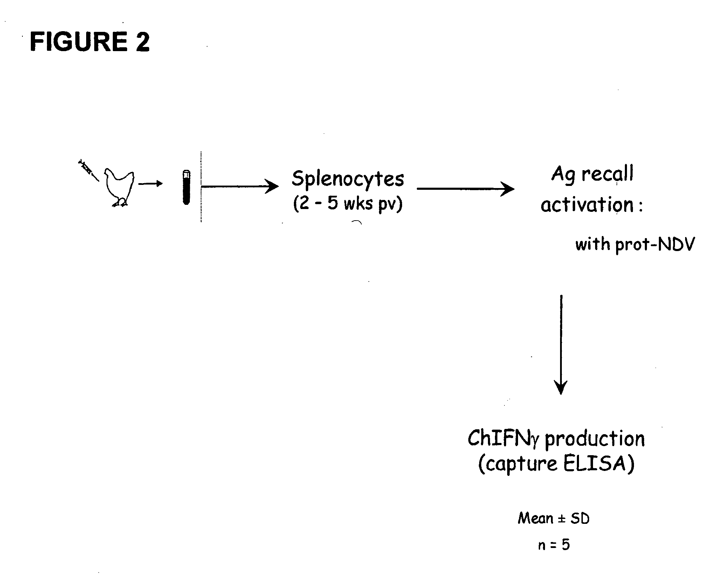

Particularly, the Applicant has demonstrated that the antigen recall activation is particularly efficient. In effect, splenocytes of vaccinated chickens were collected 2 to 5 weeks post-vaccination (p.v.) and put in contact with Newcastle disease virus protein to assess level of production of γ-interferon (ChIFNγ). Measure of chicken γ-interferon is performed by optic density (O.D.) using an Elisa kit. Level of production of ChIFNγ indicates the level of stimulation of splenocytes in vaccinated birds, thereby indicating levels of the cell-mediated immunity (Figure 2).

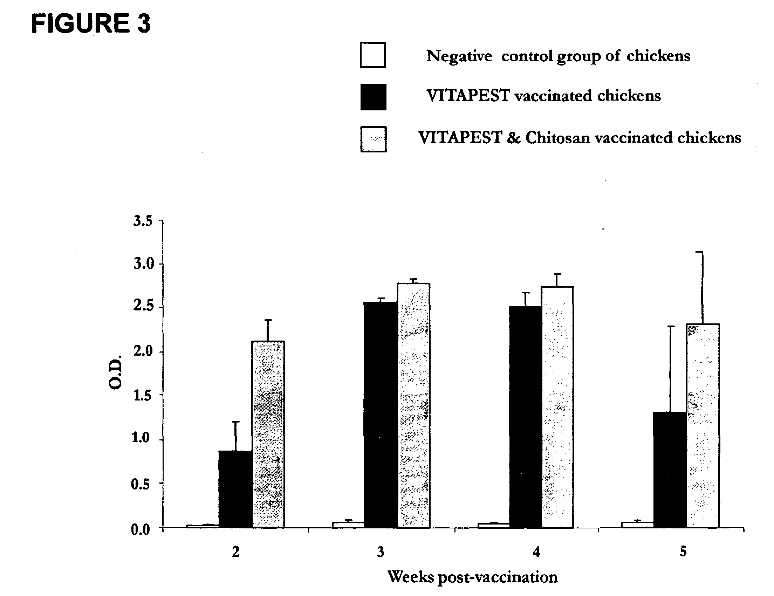

Furthermore, avian ocular vaccination according to the present invention has been demonstrated in Example 3 below to result in surprisingly high effects on the cell-mediated immunity. Without any limitations, the positive effect of chitosan on specific cell-mediated immunity has been for example evidenced against Newcastle disease virus, upon ocular administration. It has been showed that the results of production of ChIFNγ in the CEVAC^{®} VITAPEST L group and the CEVAC^{®} VITAPEST L with chitosan group (Figure 3). CEVAC^{®} VITAPEST L with chitosan group has been showed to have a higher cell-mediated immunity level than the CEVAC^{®} VITAPEST L group between the second and the fifth week post-vaccination of SPF chickens. This difference was statistically significant at week 2.

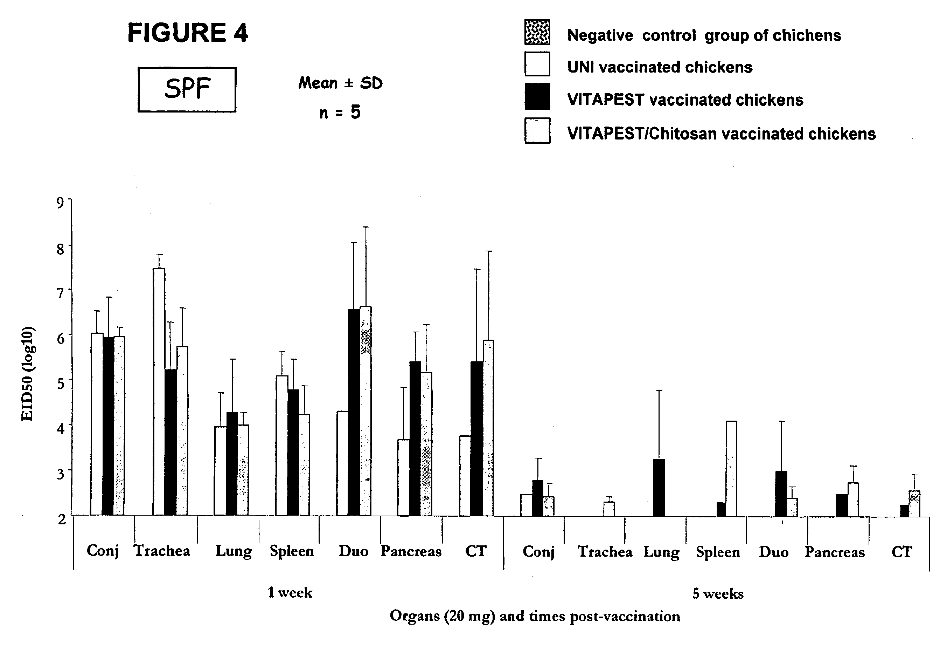

In addition, it has been demonstrated that ocular administration of vaccine with chitosan as adjuvant induced a longer persistence of the vaccine virus strain in chickens tissues, both in SPF chickens and in conventional layer chickens (Figure 4). As showed in Example 2 below, the presence of the Newcastle disease virus after ocular vaccination of SPF chickens has been assessed by real time PCR experiments on various organs, *i.e.*, conjunctiva, lung, trachea, duodenum, pancreas, caecal tonsils and spleen collected at week 1 and 5 after ocular vaccination of the chickens.

Vaccine strain virus replication has been assessed in these various organs first in SPF chickens. At the first week post-vaccination of SPF chickens, the same level of viral replication of CEVAC^{®} UNI L Vaccine virus and CEVAC^{®} VITAPEST L Vaccine virus in conjunctiva, lung and spleen has been observed. The CEVAC^{®} UNI L Vaccine virus replicated essentially in trachea, with a statistically significant difference with CEVAC^{®} VITAPEST L. The CEVAC^{®} VITAPEST L Vaccine virus replicated essentially in digestive tract, such as duodenum, pancreas and caecal tonsils, with a statistically significant difference with CEVAC^{®} UNI L. At the week 5 after ocular vaccination of the chickens, both vaccine virus strains replicated in conjunctiva but only one chicken was positive in CEVAC^{®} UNI L group. The CEVAC^{®} VITAPEST L Vaccine virus replicated in other organs as well but not CEVAC^{®} UNI L.

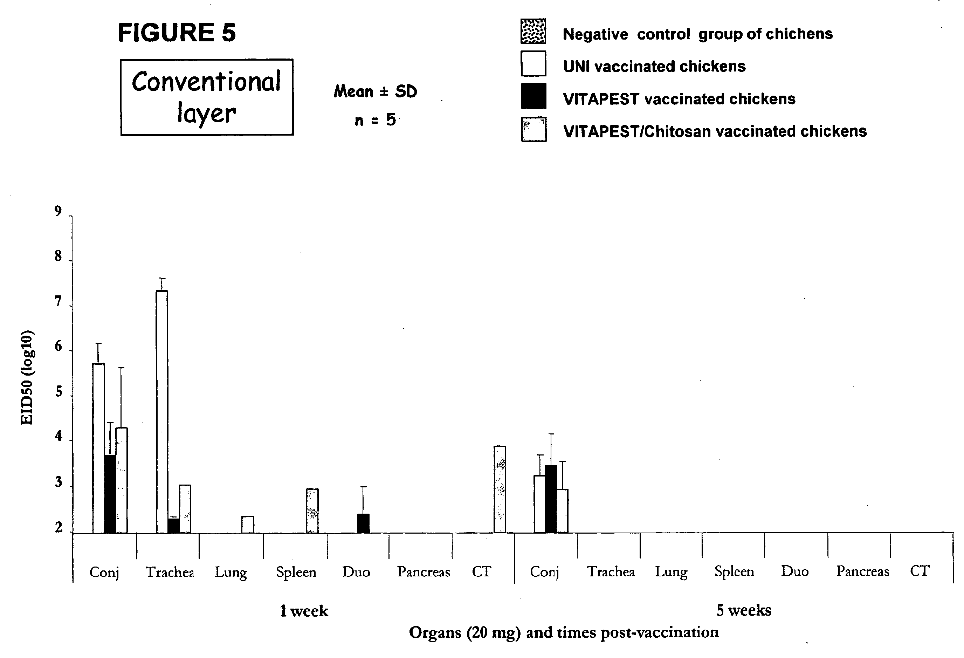

Similarly, the Applicant evidenced the persistence of the vaccine virus strain in conventional layer chickens. Particularly, it has been showed that the level of replication is higher in CEVAC^{®} UNI L group at the first week after ocular vaccination of the chickens. When chitosan is used as adjuvant, the virus is isolated in more organs and for a longer period of time. At week 5, after ocular vaccination of the chickens, the two vaccine virus strains are only isolated in conjunctiva of some vaccinated conventional layer chickens (Figure 5).

Finally, the applicant has demonstrated in Example 4 below that humoral immunity induced by various vaccines in poultry was not enhanced contrary to what is generally observed in mammals, thereby showing a specific distinctive effect of the vaccine composition with chitosan when administered to avians via the ocular route.

The invention is illustrated, but in no way limited, by the following Examples.

### EXAMPLES

### Example 1: Materials and Methods

### Example 1.1: Chickens

SPF white Leghorn and conventional layer (SSL: sex sale linked) chickens were hatched from eggs provided by Lohmann Valo (Cuxhaven, Germany) and Wijverkens hatchery (Halle, Belgium), respectively. The conventional layer eggs were obtained from a 28-week-old breeder flock which received the following ND vaccination schedule: vaccination at 4 and 8 weeks of age with NOBILIS Clone 30 (Intervet) followed by a boost at 16 weeks of age with NOBILIS Newcavac (inactivated vaccine, Intervet). After hatching, all birds were kept in biosecurity level 3 (BSL-3) isolators and animal experiments were conducted under the authorization and supervision of the Biosafety and Bioethics Committees at the Veterinary and Agrochemical Research Institute, following national and European regulations.

### Example 1.2: Vaccine, adjuvant and challenge strain

CEVAC^{®} VITAPEST L attenuated vaccine and chitosan (chitosan hydrochloride) adjuvant were provided by CEVA-PHYLAXIA Veterinary Biologicals Co Ltd (Hungary). The vaccine is based on the asymptomatic PHY.LMV.42 strain (Meszaros J. Aerosol-vaccination against Newcastle disease. Deut Tierarztl Woch 1991;98:117-164; Meszaros J, Szemeredi M, Tamasi G. Immunization of day-old chickens against Newcastle disease. Acta Vet Hung 1992; 40:121-127), belonging to genotype I (Czegledi A, Ujvari D, Somogyi E, Wehmann E, Werner O, Lomniczi B. Third genome size category of avian paramyxovirus serotype 1 (Newcastle disease virus) and evolutionary implications. Virus Res 2006; 120:36-48). Vaccine batch was reconstituted in phosphate-buffered saline (PBS buffer) to get 1 dose in 50 µl, which corresponds to ≈ 10⁶ EID₅₀/dose. The chitosan hydrochloride is a chloride salt of an unbranched binary heteropolysaccharide consisting of the two units N-acetyl-D-glucosamine and D-glucosamine. It was obtained by partial deacetylation of chitin normally leading to a degree of deacetylation of 70 per cent to 95 per cent. Chitosan was dissolved in PBS buffer at a final concentration of 0.5 % (w/v) and was used to reconstitute and dilute the vaccine to the final concentration. The Chimalhuacan NDV strain used for challenge was isolated in Mexico (Calderon NL, Galindo-Muniz F, Ortiz M, Lomniczi B, Fehervari T, Paasch LH. Thrombocytopenia in Newcastle Disease: haematological evaluation and histological study of bone marrow. Acta Vet Hung 2005;53(4):507-513) and belongs to Class II genotype V: It is a velogenic viscerotropic strain with an ICPI of 1,89. Oculo-nasal or direct ocular inoculation of 10⁵ EID₅₀ of this strain induces 100 % of mortality within 3-6 days in SPF and commercial maternally immune broiler chickens challenged within 3 to 6 weeks of age (personal observations).

### Example 1.3: Mitogens and NDV antigens

The mitogens phorbol 12-myristate 13-acetate (PMA) and ionomycin (lono) were purchased from Sigma (Belgium). NDV recall antigens were prepared from NDV La Sota strain as previously described (Lambrecht B, Gonze M, Meulemans G, van den Berg T. Assessment of the cell-mediated immune response in chickens by detection of chicken interferon-gamma in response to mitogen and recall Newcastle disease viral antigen stimulation. Avian Path 2004; 33:343-350) and named NDV glycoproteins (gp-NDV).

### Example 1.4: Measurement of vaccine strain tissue distribution and replication

Tissues samples were collected from the conjunctiva of the lower eyelid which was shown to be more susceptible to NDV (Nakamura K, Ohta Y, Abe Y, Imai K, Yamada M. Pathogenesis of conjunctivitis caused by Newcastle disease viruses in specific-pathogen-free chickens. Avian Path 2004;33(3):371-376) trachea, lung, spleen, pancreas, duodenum and caecal tonsils and kept frozen until processing. The tissue distribution and replication of CEVAC^{®} VITAPEST L vaccine strain were determined by QRRT-PCR specific to CEVAC^{®} VITAPEST L which allows detecting 10² EID₅₀ per reaction (10^{4.18} EID₅₀ per 20 mg tissue). The results were expressed as the titre of vaccine strains per 20 mg tissue. The quality of the sample and the RNA extraction procedure were validated using avian α-actin as housekeeping gene as described by Van Borm S et al., A universal avian endogenous real-time reverse transcriptase-polymerase chain reaction control and its application to avian influenza diagnosis and quantification. Avian Dis 2007;51:213-220.

### Example 1.5: Measurement of immunocompetence and NDV specific cell-mediated immunity

Immunocompetence and NDV specific cell-mediated immunity were investigated by mitogenic and NDV antigenic activation of splenic lymphocytes respectively. Briefly, spleens were aseptically removed from chickens and splenocytes were isolated and activated by mitogens (0.1 µg/ml PMA/lono), as positive control of *ex vivo* activability of splenocytes, or NDV recall antigens (gp-NDV) (1 µg/ml). Cellular responses were expressed as the optical density (O.D.) values and an O.D. equal to or greater than 0.1 was considered as evidence of significant activation.

### Example 1.6: Measurement of the NDV specific humoral and local antibody-mediated immunity

NDV specific humoral immunity was evaluated by haemagglutination inhibition (HI) test and by NDV specific IgG, IgA and IgM ELISAs. Local antibody-mediated immunity to NDV was measured in tears, lung, bile and duodenum by ELISA.

### Example 1.7: Measurement of oropharyngeal and cloacal excretion of challenge NDV strain

The oropharyngeal and cloacal cotton swabs were immersed in 1 ml of Brain Heart Infusion sampling buffer (37 g dissolved in 1 liter of distilled water) (Becton Dickinson Benelux, Belgium) supplemented with antibiotics (10 000 IU/ml of penicillin, 2 mg/ml of streptomycin, 1 mg/ml of gentamycin and 650 µg/ml of kanamycin). The swabs were stored at - 80 °C until further analysis. For analysis by quantitative real-time reverse transcription-polymerase chain reaction (QRRT-PCR), RNA was extracted from 50 µl of immersed swabs using the MagMAX 96 Al/ND viral RNA Ambion kit (Applied Biosystems, Lennik, Belgium) on a KingFisher magnetic particle processor (Thermo Scientific) according to manufacturer's protocol. Purified RNA was eluted in 50 µl of elution buffer.

The number of matrix gene copies of Chimalhuacan NDV challenge strain was determined by quantitative real-time reverse transcription-polymerase chain reaction (QRRT-PCR) as described above with minor modifications. Briefly, after an initial reverse transcription step and a hot start Taq-activation step, 50 cycles (95 °C for 15 sec, 54 °C for 34 sec, 72 °C for 10 sec) were performed on an Applied Biosystems 7500 real-time PCR cycler. Primers (M+4100 and M-4220) and MGB-Taqman probes (M+4169FAM-TAMRA) worked on the Matrix gene and were synthesized by Eurogentec (Liège, Belgium). The viral titre of each sample was determined relative to a standard curve consisting of total viral RNA of Chimalhuacan NDV strain. This standard curve was included in each run. The sensibility threshold of NDV QRRT-PCR specific to Chimalhuacan NDV strain (R² = 0.998, Efficiency = 94.17%) was determined at 10¹ EID₅₀ per reaction, based on results of standard curve. It means that QRRT-PCR reactions are completely comparable above 10²⁷ EID₅₀ per ml of swabs and quantitative comparisons possible. The results were expressed as the titre of challenge strain per ml of swabs.

Moreover, the quality of the sample and the RNA extraction procedure were validated using avian α-actin as described by Van Borm S et al. A universal avian endogenous real-time reverse transcriptase-polymerase chain reaction control and its application to avian influenza diagnosis and quantification. Avian Dis 2007;51:213-220.

### Example 1.8: Experimental design

Two similar experiments were conducted in SPF chickens (Experiments I and II). At one day of age, 105 SPF chickens were divided into three groups of 35 in BSL-3 isolators. The same day, two groups were vaccinated by oculo-nasal route with a single dose of CEVAC^{®} VITAPEST L vaccine adjuvanted or not with 0.5 % chitosan, respectively. Similar experiments are conducted by direct ocular administration (data not shown). The third group remained unvaccinated and served as negative control group. Two days post-vaccination (p.v.), chicks were humanely sacrificed to take blood, bile and duodenum. From 1 to 5 weeks p.v., at weekly intervals, tears were collected and the same animals were sampled for blood, spleen, bile and duodenum. At weeks 1 and 5, conjunctiva, trachea, lung, pancreas and caecal tonsils were additionally taken in one of the experiments. At weeks 3 and 5, lung washings were collected from additional chickens from each group after euthanasia by Nembutal (CEVA Santé Animale) injection. Five birds per group were used at each time point. In experiment II, tears, blood, spleen, bile and duodenum were sampled at 2, 3, 4 and 5 weeks p.v..

Two further experiments were also conducted in conventional layer chickens (Experiments III and IV). At one day of age, 90 conventional layer chickens were assigned to 3 groups of 30 chicks each in BSL-3 isolators. The same day, two groups were vaccinated as previously described; the third group remained unvaccinated and served as negative control group. The experiment setup of experiment III was similar to experiment I, except that lung washings were collected only at 5 weeks p.v. In experiment IV, tears, blood, spleen, bile and duodenum were sampled at 2, 3, 4 and 5 weeks p.v.. At 5 weeks of age, 10 chickens from each group were challenged with 10⁵ EID₅₀/200 µl of Chimalhuacan NDV strain by oculo-nasal route. The birds were individually identified. After challenge, chickens were monitored daily for clinical symptoms (oedema of the head, depression, prostration and nervous signs) and mortality. Oropharyngeal and cloacal swabs were taken at 2, 4, 7 and 10 days post-challenge (p.ch.). At 11 days p.ch., surviving chickens were euthanized and blood samples were collected. Spleen and duodenum were taken from 5 chickens per group.

### Example 1.9: Statistical analysis

Statistical analyses were performed as previously described using Minitab 13 and STATA 10 software (statistical programmes for Windows 2000) and differences were considered as significant at *P* < *0.05.*

### Example 2: Tissue distribution and replication of vaccine strain

All organ samples showed positive amplification signal (Ct < 40) for the α-actin endogenous amplification control, validating their adequate quality and RNA purity.

The CEVAC^{®} VITAPEST L virus was detected in all organ samples of the vaccinated SPF chickens at 1 week p.v. (Table 1). At 5 weeks p.v., the vaccine strain was only detected in the conjunctiva, lung, spleen, duodenum, pancreas and caecal tonsils of some chickens. When chitosan was used, the vaccine strain was more frequently detected; however, there was no statistical difference among the viral titres. One week after vaccination of one-day-old conventional layer chickens, the CEVAC^{®} VITAPEST L vaccine strains were present in several samples of conjunctiva, trachea and duodenum (Table 1). When the vaccine was adjuvanted with chitosan, it was also detected in some lung, spleen and caecal tonsil samples. Nevertheless, in the vaccinated conventional layers, the detection frequencies and the titres were lower than those found in vaccinated SPF chickens. At 5 weeks p.v., replication of the vaccine virus could still be detected in the conjunctiva.

**Table 1: Detection of vaccine virus after vaccination of day-old SPF and conventional layer chickens with CEVAC^{®} VITAPEST L vaccine with or without chitosan adjuvant (experiment I and III, respectively).**

| **Exp.** | **Week p.v** | **Tissues** | **Negative** | **Groups ^{a} VITAPEST** | **VITAPEST+ Chitosan** |
|---|---|---|---|---|---|
| 1 (SPF) | 1 | Conjunctiva ^{b c} | 0/5 ^{B} | 5/5 ^{A} | 5/5 ^{A} |
| | | | <4.18 | 8.12±0.91 ^{A} | 8.13±0.22 ^{A} |
| | | Trachea | 0/5 ^{B} | 5/5 ^{A} | 5/5 ^{A} |
| | | | <4.18 | 7.89±1.07 ^{A} | 7.93±0.84 ^{A} |
| | | Lung | 0/5 ^{B} | 5/5 ^{A} | 5/5 ^{A} |
| | | | <4.18 | 6.45±1.19 ^{A} | 6.18±0.27 ^{A} |
| | | Spleen | 0/5 ^{A} | 4/5 ^{A} | 4/5 ^{A} |
| | | | <4.18 | 6.97±0.68 ^{A} | 6.41±0.65 ^{A} |
| | | Duodenum | 0/5 ^{B} | 5/5 ^{A} | 5/5 ^{A} |
| | | | <4.18 | 8.77±1.47 | 8.83±1.76 |
| | | Pancreas | 0 / 5 ^{B} | 5 / 5 ^{A} | 5 / 5 ^{A} |
| | | | < 4.18 | 7.61±0.64^{A} | 7.36±1.07^{A} |
| | | Caecal tonsils | 0 / 5 ^{B} | 3 / 5 ^{AB} | 5 / 5 ^{A} |
| | | | < 4.18 | 7.60±2.06^{A} | 8.08±1.9^{A} |
| | 5 | Conjunctiva | 0 / 5 ^{B} | 5 / 5 ^{A} | 4 / 5 ^{A} |
| | | | < 4..18 | 4.99 ± 0.48^{A} | 4.63±0.30^{A} |
| | | Trachea | 0 / 5 ^{A} | 0 / 5 ^{A} | 2 / 5 ^{A} |
| | | | < 4.18 | < 4.18 | 4.51±0.11 |
| | | Lung | 0 / 5 ^{A} | 2 / 5 ^{A} | 0 / 5 ^{A} |
| | | | < 4.18 | 5.46±1.50 | <4.18 |
| | | Spleen | 0 / 5 ^{A} | 1 / 5 ^{A} | 1 / 5 ^{A} |
| | | | < 4.18 | 4.50 | 6.29 |
| | | Duodenum | 0 / 5 ^{A} | 2 / 5 ^{A} | 3 / 5 ^{A} |
| | | | < 4.18 | 5.19±1.09^{A} | 5.60±0.26 ^{A} |
| | | Pancreas | 0 / 5 ^{A} | 1 / 5 ^{A} | 3 / 5 ^{A} |
| | | | < 4.18 | 4.69 | 4.95±0.37 |
| | | Caecal tonsils | 0 / 5 ^{A} | 1 / 5 ^{A} | 3 / 5 ^{A} |
| | | | < 4.18 | 4.45 | 4.79±0.34 |
| III (conv.) | 1 | Conjunctiva ^{b c} | 0 / 5 ^{A} | 4 / 5 ^{A} | 4 / 5^{A} |
| | | | < 2 | 5,90±0,73^{A} | 6,51±1,33 ^{A} |
| | | Trachea | 0 / 5 ^{A} | 3 / 5 ^{A} | 1 / 5 ^{A} |
| | | | < 2 | 4,49±0,04 | 5,24 |
| | | Lung | 0 / 5 ^{A} | 0 / 5 ^{A} | 1 / 5 ^{A} |
| | | | < 2 | < 2 | 4,57 |
| | | Spleen | 0 / 5 ^{A} | 0 / 5 ^{A} | 1 / 5 ^{A} |
| | | | <2 | < 2 | 5,16 |
| | | Duodenum | 0 / 5 ^{A} | 2 / 5 ^{A} | 0 / 5 ^{A} |
| | | | < 2 | 4,62±0,58 | < 2 |
| | | Pancreas | 0 / 5 ^{A} | 0 / 5 ^{A} | 0 / 5 ^{A} |
| | | | <2 | < 2 | < 2 |
| | | Caecal tonsils | 0 / 5 ^{A} | 0 / 5 ^{A} | 1 / 5 ^{A} |
| | | | < 2 | < 2 | 6,10 |
| | 5 | Conjunctiva | 0 / 5 ^{A} | 3 / 5 ^{A} | 4 / 5^{A} |
| | | | < 2 | 5,66±0,69 ^{A} | 5,13±0,62^{A} |
| | | Trachea | 0 / 5 ^{A} | 0 / 5 ^{A} | 0 / 5 ^{A} |
| | | | < 2 | < 2 | < 2 |
| | | Lung | 0 / 5^{A} | 0 / 5 ^{A} | 0 / 5 ^{A} |
| | | | < 2 | < 2 | < 2 |
| | | Spleen | 0 / 5 ^{A} | 0 / 5 ^{A} | 0 / 5 ^{A} |
| | | | < 2 | < 2 | < 2 |
| | | Duodenum | 0 / 5 ^{A} | 0 / 5 ^{A} | 0 / 5 ^{A} |
| | | | < 2 | < 2 | < 2 |
| | | Pancreas | 0 / 5 ^{A} | 0 / 5 ^{A} | 0 / 5 ^{A} |
| | | | < 2 | < 2 | < 2 |
| | | Caecal tonsils | 0 / 5 ^{A} | 0 / 5 ^{A} | 0 / 5 ^{A} |
| | | | < 2 | < 2 | |

| | | | | | |
|---|---|---|---|---|---|
| ^{a} Data were determined by QRRT-PCR on 20 mg tissue at the specified time p.v.. Data with no common superscript differ significantly for this tissue (*P* < *0.05* or *0.017* with Bonferroni correction). The cut-off of QRRT-PCR specific to CEVAC^{®} VITAPEST L vaccine strain was determined as 10^{4.18} per 20 mg tissue. ^{b} Data represent frequency (number positive / total tested) of virus detection in 20 mg tissue. ^{c} Data represent mean ± standard deviation of log₁₀ EID₅₀ of NDV in 20 mg tissue of positive chickens: | | | | | |

### Example 3: Immunocompetence and antigen-specific cell-mediated immunity

The results obtained at the different sampling time points on splenocytes activation with PMA/lono mitogens showed that all SPF and conventional layer chickens in each group were similarly immunocompetent in each of four experiments (O.D. > 0.1), validating the spleen cells activability. Indeed, chitosan did not affect the cellular immune response, as evidenced by the absence of any significant difference between the two vaccinated groups in experiments I (Figure 6A) and IV (Figure 6B) on SPF and conventional layer chickens, respectively. This unchanged immunocompetence, when live ND vaccine was co-administrated with chitosan, was confirmed in the experiments II and III (data not shown).

In SPF chicks (experiment I), the CEVAC^{®} VITAPEST L strain was able to induce a measurable activation of NDV specific cell-mediated immunity within the 1^{st} week p.v. and the vaccinated group differed significantly (*P* < *0.05*) from the unvaccinated control group from the 2^{nd} week onward (Figure 6A). When supplemented with chitosan, oculo-nasal immunization with CEVAC^{®} VITAPEST L vaccine generated a significantly higher antigen-specific cellular immune response from week 1 to week 4 p.v. (P < 0.05), as demonstrated by increased ChIFNγ production of splenocytes. The positive effect of chitosan on specific cell-mediated immunity in SPF chickens was confirmed in experiment II (data not shown).

In conventional layer chicks (experiment IV), NDV specific cell-mediated immunity was observed in both vaccinated groups from the 3^{rd} week p.v. (P < 0.05) (Figure 6B). This NDV specific cellular response is higher, although not significantly, when chitosan is co-administrated with the ND live vaccine, compared with CEVAC^{®} VITAPEST L vaccine inoculated alone. The same result was obtained in experiment III (data not shown).

### Example 4: Humoral and local antibody-mediated immunity

Induction of humoral immunity in the vaccinated SPF chickens was recorded since the 1^{st} week p.v. by specific IgM ELISA but only from the 2^{nd} week p.v. by haemagglutination inhibition test as well as by IgG and IgA specific ELISAs (Table 2), with titres statistically different from the unvaccinated chickens. The profile of the NDV specific HI and IgG antibody titres obtained in conventional layer chickens corresponds to the decline of passive maternally-derived immunity until the 4^{th} week of age and a progressive active vaccine-induced primary immune response detectable from the 5^{th} week p.v. (Table 2). Neither NDV specific IgA nor IgM could be detected in the serum of conventional animals (data not shown). The chitosan showed no adjuvant effect on the humoral immune-response in neither SPF nor conventional layer chickens, as demonstrated by the absence of any statistically significant differences in the titres between the two vaccinated groups.

**Table 2: Humoral immunity after vaccination of day-old SPF and conventional layer chickens with CEVAC^{®} VITAPEST L vaccine with or without chitosan adjuvant (experiments I and III, respectively).**

| Exp. | Humoral response | Times p.v. | Negative | Groups VITAPEST | VITAPEST + Chitosan |
|---|---|---|---|---|---|
| I (SPF) | HI | 2 days | 2.00 ± 0.00^{A} | 2.20 ± 0.45^{A} | 2.50 ± 0.71^{A} |
| | | 1 week | 2.50 ± 0.58^{A} | 2.67 ± 0.58^{A} | 2.60 ± 0.55^{A} |
| | | 2 weeks | 2.40 ± 0.55^{B} | 8.40 ± 2.30^{A} | 7.80 ± 3.49^{A} |
| | | 3 weeks | 2.00 ± 0.00^{B} | 8.00 ± 2.00^{A} | 7.00 ± 1.22^{A} |
| | | 4 weeks | 2.20 ± 0.45^{B} | 7.80 ± 0.84^{A} | 8.00 ± 1.22^{A} |
| | | 5 weeks | 2.00 ± 0.00^{B} | 7.40 ± 1.52^{A} | 6.40 ± 1.34^{A} |
| | IgG | 2 days | 0.080 ± 0.050^{A} | 0.085 ± 0.048^{A} | 0.061 ± 0.050^{A} |
| | | 1 week | 0.053 ± 0.026^{A} | 0.085 ± 0.040^{A} | 0.034 ± 0.006^{A} |
| | | 2 weeks | 0.036 ± 0.006^{B} | 1.297 ± 0.284^{A} | 1.255 ± 0.305^{A} |
| | | 3 weeks | 0.032 ± 0.007^{B} | 1.559 ± 0.081^{A} | 1.410 ± 0.070^{A} |
| | | 4 weeks | 0.054 ± 0.026^{B} | 1.605 ± 0.102^{A} | 1.548 ± 0.125^{A} |
| | | 5 weeks | 0.078 ± 0.015^{B} | 1.354 ± 0.462^{A} | 1.527 ± 0.267^{A} |
| | IgM | 2 days | 0.073 ± 0.018^{A} | 0.090 ± 0.032^{A} | 0.079 ± 0.008^{A} |
| | | 1 week | 0.126 ± 0.016^{B} | 0.748 ± 0.384^{A} | 0.559 ± 0.194^{A} |
| | | 2 weeks | 0.157 ± 0.043^{B} | 1.557 ± 0.233^{A} | 1.422 ± 0.251^{A} |
| | | 3 weeks | 0.169 ± 0.043^{B} | 0.950 ± 0.392^{A} | 0.891 ± 0.330^{A} |
| | | 4 weeks | 0.309 ± 0.073^{B} | 1.227 ± 0.153^{A} | 0.872 ± 0.254^{AB} |
| | | 5 weeks | 0.381 ± 0.149^{B} | 1.248 ± 0.455^{A} | 0.915 ± 0.288^{AB} |
| | IgA | 2 days | 0.057 ± 0.010^{A} | 0.054 ± 0.007^{A} | 0.054 ± 0.003^{A} |
| | | 1 week | 0.057 ± 0.006^{B} | 0.211 ± 0.098^{A} | 0.160 ± 0.088^{AB} |
| | | 2 weeks | 0.112 ± 0.039^{C} | 1.031 ± 0.285^{A} | 0.565 ± 0.174^{B} |
| | | 3 weeks | 0.103 ± 0.031^{B} | 0.429 ± 0.213^{A} | 0.471 ± 0.148^{A} |
| | | 4 weeks | 0.140 ± 0.065^{A} | 0.420 ± 0.218^{A} | 0.447 ± 0.519^{A} |
| | | 5 weeks | 0.140 ± 0.029^{A} | 0.292 ± 0.146^{A} | 0.236 ± 0.146^{A} |
| III (conv.) | HI | 2 days | 10.40 ± 1.52^{A} | 9.60 ± 0.89^{A} | 10.40 ± 0.55^{A} |
| | | 1 week | 9.80 ± 1.30^{A} | 920 ± 0.45^{A} | 9.20 ± 0.84^{A} |
| | | 2 weeks | 6.80 ± 1.30^{A} | 7.20 ± 0.84^{A} | 7.40 ± 1.14^{A} |
| | | 3 weeks | 5.40 ± 0.89^{B} | 6.80 ± 0.45^{A} | 5.20 ± 0.45^{B} |
| | | 4 weeks | 4.20 ± 1.30^{A} | 4.60 ± 0.89^{A} | 5.20 ± 0.84^{A} |
| | | 5 weeks | 3.00 ± 0.00^{B} | 4.80 ± 0.45^{A} | 5.60 ± 0.55^{A} |
| | IgG | 2 days | 1.637 ± 0.045^{A} | 1.421 ±0.025^{A} | 1.555 ± 0.080^{A} |
| | | 1 week | 1.565 ± 0.094^{A} | 1.551 ±0.100^{A} | 1.503±0.108^{A} |
| | | 2 weeks | 1.505 ±0.121^{A} | 1.434±0.085^{A} | 1.434±0.070^{A} |
| | | 3 weeks | 1.250±0.377^{A} | 1.364±0.097^{A} | 1.241±0.137^{A} |
| | | 4 weeks | 0.659±0.556^{A} | 0.766±0.383^{A} | 0.931±0.506^{A} |
| | | 5 weeks | 0.143±0.212^{B} | 1.036±0.215^{A} | 1.080±0.160^{A} |

| | | | | | |
|---|---|---|---|---|---|
| Data represent mean ± standard deviation of reciprocal log₂ of serum dilution and absorbance values determined by HI and ELISA tests, respectively, at specified time p.v. (n = 5). Serum samples were diluted 1:100. Mean ± standard deviation at time points with no common superscript differ significantly (*P* < *0.05*). | | | | | |

The effect of chitosan on lachrymal antibody-mediated immunity of SPF (Figure 7A) and conventional layer chickens (Figure 7B) was highly variable and the differences between the chickens vaccinated with CEVAC^{®} VITAPEST L with or without (w/o) chitosan were not statistically significant at each time point. Similar variation in the effects of the chitosan adjuvant was noted for lung-associated, biliary and duodenal antibody-mediated immunity of SPF chickens (Table 3, Figures 8A and 8A, respectively). In conventional layer chickens, NDV specific IgG antibody of maternal origin was detected on the 1^{st} week in bile (Figure 9B) and from the 1^{st} until the 5^{th} week of age in duodenum (Figure 9B). On 5 weeks p.v., the duodenal IgG response was significantly higher in the vaccinated groups, indicating a progressive active vaccine-induced primary immune response in the digestive tract. The co-administration of chitosan with live ND vaccine has no significant effect on the duodenal antibody-mediated immunity.

**Table 3 : Pulmonary antibody-mediated immunity after vaccination of day-old SPF chickens with CEVAC^{®} VITAPEST L vaccine with or without chitosan adjuvant (experiment I).**

| Humoral response | Week p.v. | Negative | Groups VlTAPEST | VlTAPEST + Chitosan |
|---|---|---|---|---|
| IgA | 3 | 0.258±0.012^{A} | 0.433±0.327^{A} | 0.335±0.040^{A} |
| | 5 | 0.319±0.078^{A} | 0.505±0.191^{A} | 0.662±0,487^{A} |
| IgG | 3 | 0.098±0.011^{A} | 0.619±0.304^{A} | 0.429±0.059^{A} |
| | 5 | 0.055±0.013^{A} | 0.300±0.411^{A} | 0.358±0.393^{A} |
| IgM | 3 | 0.323±0.014^{A} | 0.385±0.206^{A} | 0.282±0.017^{A} |
| | 5 | 0.328±0.043^{A} | 0.508±0.127^{A} | 0.399±0.165^{A} |

| | | | | |
|---|---|---|---|---|
| Data represent mean ± standard deviation of absorbance values determined by ELISA at specified time p.v. (n = 5). Lung washing samples were undiluted for IgA/G and IgM, respectively. Mean ± standard deviation at time points with no common superscript differ significantly (*P* < *0.05*). | | | | |

This unchanged systemic and local antibody response to live ND vaccine co-administrated with chitosan was confirmed both in SPF and conventional layer chickens in the repeated experiments II and IV (data not shown).

### Example 5: Protection and re-excretion after challenge

All challenged chickens in the unvaccinated group showed clinical signs, which started at 3 days post-challenge (d.p.ch.) and died between 4 and 6 d.p.ch.. Two animals in the CEVAC^{®} VITAPEST L vaccinated group showed clinical signs and one died at 7 d.p.ch.. Chickens vaccinated with CEVAC^{®} VITAPEST L co-administrated with chitosan showed neither specific morbidity nor mortality during the experiment.

Shedding of challenge virus started at 2 d.p.ch. in the control group and all oropharyngeal and cloacal swabs were positive at 4 d.p.ch. (Table 4). At each time point, shedding by the oropharyngeal and cloacal routes was reduced by vaccination. In addition, the number of excreting birds in the group receiving the ND live vaccine co-administrated with chitosan was reduced compared to the group receiving the vaccine alone. Excretion was nearly finished at 7 d.p. ch. and completely stopped at 10 d.p.ch..

**Table 4: Shedding of Chimalhuacan NDV strain after challenge of vaccinated conventional layer chickens with CEVAC^{®} VITAPEST L vaccine with or without chitosan adjuvant (experiment IV).**

| Days p. ch. | Tissues | Negative | Groups^{a} VITAPEST | VITAPEST + Chitosan |
|---|---|---|---|---|
| 2 | Oropharynx ^{b c} | 7 / 10 ^{B} | 1 / 10 ^{AB} | 0 / 10 ^{A} |
| | | 3.74±0.76 | 2.79 | < 2.70 |
| | Cloaca | 1 / 10 ^{A} | 0 / 10 ^{A} | 0 / 10 ^{A} |
| | | 3.57 | < 2.70 | < 2.70 |
| 4 | Oropharynx | 10 / 10^{A} | 6 / 10 ^{A} | 5 / 10 ^{A} |
| | | 4.56 ± 0.50 ^{A} | 3.99 ± 0.68^{A} | 3.79±0.12^{A} |
| | Cloaca | 10 / 10 ^{A} | 8 / 10^{A} | 6 / 10^{A} |
| | | 5.11±0.78^{A} | 3.72 ± 0.63^{B} | 3.78±0.46^{B} |
| 7 | Oropharynx | N.D. | 1 / 9 ^{A} | 1 / 10^{A} |
| | | | 4.65 | 4.07 |
| | Cloaca | N.D. | 1 / 9 ^{A} | 0 / 10 ^{A} |
| | | | 3.47 | < 2.70 |
| 10 | Oropharynx | N.D. | 0 / 9 ^{A} | 0 / 10 ^{A} |
| | | | < 2.70 | < 2.70 |
| | Cloaca | N.D. | 0 / 9 ^{A} | 0 / 10 ^{A} |
| | | | < 2.70 | < 2.70 |

| | | | | |
|---|---|---|---|---|
| ^{a} Data are determined by QRRT-PCR on 1 ml of swabs taken at specified time p.ch.. Data with no common superscript differ significantly for this swab (P < 0.05 or 0.017 with Bonferroni correction). The cut-off of QRRT-PCR specific to Chimalhuacan NDV strain was determined at 10^{2.7} per ml swabs. The total numbers of chickens tested were reduced with time because of specific mortality (see the results section for details). N.D. = not determined, due to specific mortality. ^{b} Data represent frequency (number positive / total tested) of virus detection in 1 ml swabs. | | | | |

## Claims

1. A composition comprising a live attenuated Newcastle Disease Virus and chitosan for use to immunize or vaccinate avian species by ocular administration via eye-drops, a spray or an aerosol.

2. The composition of claim 1, for use to immunize or vaccinate an avian species selected from raised or domesticated poultry or fowl, including chickens, turkeys, geese, ducks, pheasants, quail, or pigeons.

3. The composition of any one of claims 1 to 2 for increasing cell-mediated immunity in avian species and persistence of the vaccine in avian tissues.

4. Use of chitosan and a live attenuated Newcastle Disease Virus for the preparation of a vaccine composition for immunizing or vaccinating avian species by ocular administration via eye-drops, a spray or an aerosol.

5. An avian vaccination kit comprising, in a dispensing device selected from eye-drops, a spray or an aerosol, a live attenuated Newcastle Disease Virus and chitosan.

## Patentansprüche

1. Eine Zusammensetzung umfassend einen attenuierten Lebendvirus der Newcastle-Krankheit und Chitosan zur Verwendung, um Vogelspezies durch okulare Verabreichung mittels Augentropfen, einem Spray oder einem Aerosol zu immunisieren oder zu impfen.

2. Die Zusammensetzung nach Anspruch 1 zur Verwendung, um eine Vogelspezies ausgewählt aus aufgezogenen oder gezähmten Geflügel oder Federvieh, einschließlich Hühnchen, Puten, Gänsen, Enten, Fasanen, Wachtel oder Tauben, zu immunisieren oder zu impfen.

3. Die Zusammensetzung nach einem der Ansprüche 1 bis 2 zur Erhöhung der zellvermittelten Immunität in Vogelspezies und der Beständigkeit des Impfstoffs in Vogelgeweben.

4. Verwendung von Chitosan und einem attenuierten Lebendvirus der Newcastle-Krankheit für die Herstellung einer Impfstoffzusammensetzung zum Immunisieren und Impfen von Vogelspezies durch okulare Verabreichung durch Augentropfen, einem Spray oder einem Aerosol.

5. Ein Vogelimpfstoffkit umfassend in einer Dosierungseinheit, ausgewählt aus Augentropfen, einem Spray oder einem Aerosol, einem attenuierten Lebendvirus der Newcastle-Krankheit und Chitosan.

## Revendications

1. Composition comprenant un Virus de la Maladie de Newcastle vivant atténué et du chitosan pour une utilisation pour immuniser ou vacciner des espèces aviaires par administration oculaire par gouttes, spray ou aérosol.

2. Composition selon la revendication 1, pour une utilisation pour immuniser ou vacciner une espèce aviaire choisie parmi la volaille d'élevage ou domestiquée, y compris des poulets, dindes, oies, canards, faisans, cailles, ou pigeons.

3. Composition selon l'une quelconque des revendications 1 à 2, pour augmenter l'immunité cellulaire chez les espèces aviaires et la persistance du vaccin dans les tissus aviaires.

4. Utilisation de chitosan et d'un Virus de la Maladie de Newcastle vivant atténué pour la préparation d'une composition vaccinale pour immuniser ou vacciner des espèces aviaires par administration oculaire par gouttes, spray ou aérosol.

5. Kit de vaccination aviaire comprenant, dans un dispositif de dispersion choisi parmi les gouttes, un spray ou un aérosol, un Virus de la Maladie de Newcastle vivant atténué et du chitosan.
